(19) Europäisches Patentamt
European Patent Office
Office européen des brevets

(11) Veröffentlichungsnummer: **0 475 122 A1**

(12) # EUROPÄISCHE PATENTANMELDUNG

(21) Anmeldenummer: **91113811.3**

(22) Anmeldetag: **17.08.91**

(51) Int. Cl.[5]: **C07C 29/40**, C07C 33/50, C07D 303/08, C07F 3/02

(30) Priorität: **31.08.90 DE 4027608**

(43) Veröffentlichungstag der Anmeldung: **18.03.92 Patentblatt 92/12**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Rosen, Winfried, Dr.**
**Pahlkestrasse 5**
**W-5600 Wuppertal 1(DE)**
Erfinder: **Fiedler, Paul-Christian, Dr.**
**Rybniker Strasse 8**
**W-5000 Köln 80(DE)**
Erfinder: **Blume, Götz, Dr.**
**Bebelstrasse 41**
**W-3450 Holzminden(DE)**

(54) **Verfahren zur Herstellung von Zwischenprodukten.**

(57) Nach einem neuen Verfahren lassen sich 1-Chlor-2-(1-chlor-cyclopropyl)-3-(2-chlorphenyl)-propan-2-ol und/oder 2-(1-Chlorcyclopropyl)-2-(2-chlor-benzyl)-oxiran herstellen, indem man

a) 2-Chlor-benzylchorid mit zerkleinertem Magnesium, gegebenenfalls in Gegenwart eines Katalysators, in Gegenwart eines Gemisches von Toluol und Tetrahydrofuran, in dem das Verhältnis von Toluol zu Tetrahydrofuran zwischen 65:35 und 95:5 Gewichtsteilen liegt, bei Temperaturen zwischen 0 °C und 100 °C umsetzt, wobei das Verhältnis von 2-Chlor-benzylchlorid zu Tetrahydrofuran so bemessen ist, daß auf 1 Mol an 2-Chlor-benzylchlorid zwischen 1 und 3 Mol an Tetrahydrofuran vorhanden sind, anschließend gegebenenfalls noch vorhandenes Magnesium abtrennt und

b) das entstandene Grignard-Reagenz mit 1-Chlor-1-chloracetyl-cyclopropan in Gegenwart eines Gemisches von Toluol und Tetrahydrofuran, in dem das Verhältnis von Toluol zu Tetrahydrofuran zwischen 65:35 und 95:5 Gewichtsteilen liegt, sowie gegebenenfalls in Gegenwart eines zusätzlichen inerten Verdünnungsmittels bei Temperaturen zwischen 0 °C und 100 °C umsetzt.

EP 0 475 122 A1

Die vorliegende Erfindung betrifft ein neues Verfahren zur Herstellung von 1-Chlor-2-(1-chlor-cyclopropyl)-3-(2-chlor-phenyl)-propan-2-ol und/oder 2-(1-Chlor-cyclopropyl)-2-(2-chlor-benzyl)-oxiran, die als Zwischenprodukte zur Synthese des fungizid wirksamen 2-(1-Chlor-cyclopropyl)-1-(2-chlor-phenyl)-3-(1,2,4-triazol-1-yl)-propan-2-ols verwendet werden können.

Es ist bereits bekannt geworden, daß sich bestimmte, als Zwischenprodukte zur Synthese von Azolyl-Derivaten geeignete Cyclopropylhydrine und Cyclopropyl-oxirane herstellen lassen, indem man Benzyl-Grignard-Verbindungen mit Halogenacetyl-cyclopropyl-Derivaten zur Reaktion bringt (vergl. EP-OS 0 297 345). So erhält man zum Beispiel 1-Chlor-2-(1-chlor-cyclopropyl)-3-(4-fluor-phenyl)-propan-2-ol dadurch, daß man 4-Fluor-benzylbromid mit Magnesium-Spänen in Gegenwart von Diethylether umsetzt und die entstehende Grignard-Verbindung mit 1-Chlor-1-chloracetyl-cyclopropan in Gegenwart von Diethylether weiter reagieren läßt. Nachteilig an diesem Verfahren ist jedoch, daß eine solche Umsetzung in Diethylether als Verdünnungsmittel aus Sicherheitsgründen in technischem Maßstab extrem schwierig durchführbar ist.

Weiterhin ist schon bekannt, daß sich Organo-Magnesium-Verbindungen in manchen Fällen in Gegenwart von Ethern, wie Tetrahydrofuran, und von aromatischen Kohlenwasserstoffen, wie Toluol, oder deren Gemischen als Verdünnungsmittel herstellen lassen (vergl. US-PS 3 388 179). Eine breite Anwendbarkeit dieser Methode ist aber bisher noch nicht beschrieben worden.

Es wurde nun gefunden, daß man 1-Chlor-2-(1-chlor-cyclopropyl)-3-(2-chlor-phenyl)-propan-2-ol der Formel

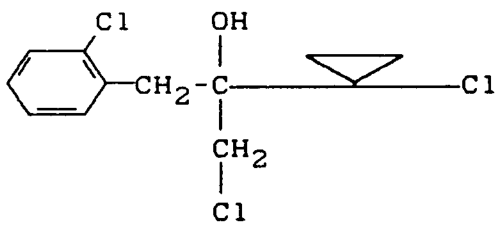

(Ia)

und/oder 2-(1-Chlor-cyclopropyl)-2-(2-chlor-benzyl)-oxiran der Formel

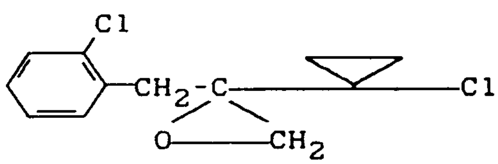

(Ib)

erhält, indem man

a) 2-Chlor-benzylchlorid der Formel

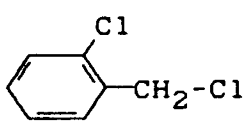

(II)

mit zerkleinertem Magnesium, gegebenenfalls in Gegenwart eines Katalysators, in Gegenwart eines Gemisches von Toluol und Tetrahydrofuran, in dem das Verhältnis von Toluol zu Tetrahydrofuran zwischen 65:35 und 95:5 Gewichtsteilen liegt, bei Temperaturen zwischen 0°C und 100°C umsetzt, wobei das Verhältnis von 2-Chlor-benzylchlorid der Formel (II) zu Tetrahydrofuran so bemessen ist, daß auf 1 Mol an 2-Chlor-benzylchlorid der Formel (II) zwischen 1 und 3 Mol an Tetrahydrofuran vorhanden sind,

anschließend gegebenenfalls noch vorhandenes Magnesium abtrennt und

b) das entstandene Grignard-Reagenz der Formel

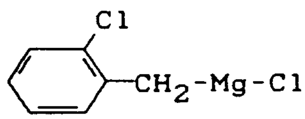

(III)

mit 1-Chlor-1-chloracetyl-cyclopropan der Formel

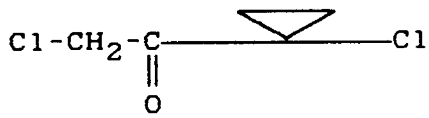

(IV)

in Gegenwart eines Gemisches von Toluol und Tetrahydrofuran, in dem das Verhältnis von Toluol zu Tetrahydrofuran zwischen 65:35 und 95:5 Gewichtsteilen liegt, sowie gegebenenfalls in Gegenwart eines zusätzlichen inerten Verdünnungsmittels bei Temperaturen zwischen 0 ° C und 100 ° C umsetzt.

Es ist als äußerst überraschend zu bezeichnen, daß sich die Zwischenprodukte der Formeln (Ia) und (Ib) nach dem erfindungsgemäßen Verfahren in sehr hoher Ausbeute herstellen lassen, während sie bei der Durchführung der Reaktion in Gegenwart von Tetrahydrofuran als alleinigem Verdünnungsmittel, oder von anderen technisch verwendbaren Ethern oder in Gegenwart von solchen Toluol/Tetrahydrofuran-Gemischen, die nur wenig Toluol enthalten, nur in geringen Ausbeuten anfallen. Unerwartet ist auch, daß die gewünschten Produkte beim Einsatz von 2-Chlor-benzylchlorid der Formel (II) in wesentlich besserer Ausbeute entstehen als bei Verwendung des entsprechenden 2-Chlor-benzylbromids.

Das erfindungsgemäße Verfahren zeichnet sich durch eine Reihe von Vorteilen aus. So läßt sich die Grignard-Reaktion, bei der bestimmte Gemische aus Toluol und Tetrahydrofuran als Verdünnungsmittel dienen, auch im technischen Maßstab ohne Schwierigkeiten durchführen. Günstig ist außerdem, daß die gewünschten Produkte in sehr hohen Ausbeuten und guter Reinheit erhalten werden. Ein weiterer Vorteil des erfindungsgemäßen Verfahrens besteht darin, daß es nicht nur diskontinuierlich, sondern auch kontinuierlich durchgeführt werden kann.

Der Verlauf des erfindungsgemäßen Verfahrens kann durch das folgende Formelschema veranschaulicht werden:

a)

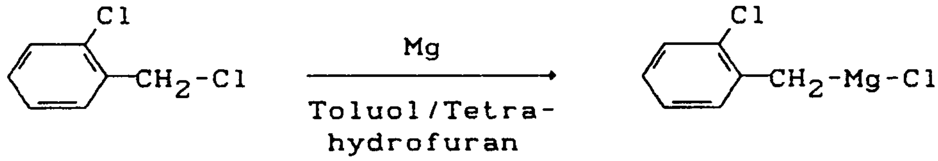


b)

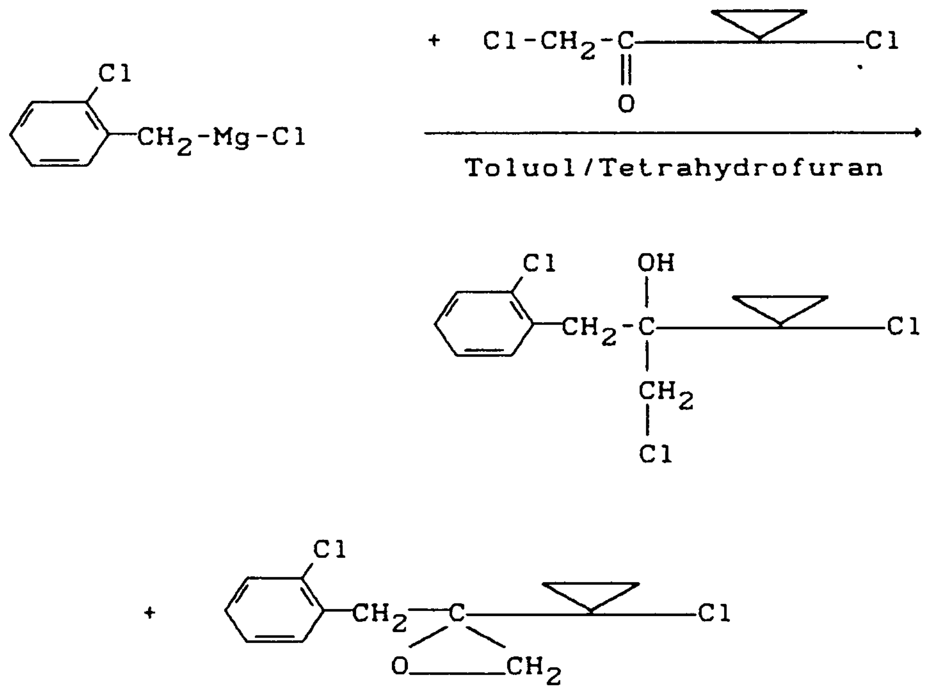


Das bei der Durchführung des erfindungsgemäßen Verfahrens als Ausgangssubstanz benötigte 2-Chlorbenzylchlorid der Formel (II) ist bekannt.

Das bei der Durchführung des erfindungsgemäßen Verfahrens in der zweiten Stufe als Reaktionskomponente benötigte 1-Chlor-1-chloracetyl-cyclopropan der Formel (IV) ist ebenfalls bekannt (vergl. EP-OS 0 297 345).

Das Magnesium wird bei der Durchführung der ersten Stufe des erfindungsgemäßen Verfahrens in zerkleinerter Form eingesetzt. Vorzugsweise verwendbar sind Magnesium-Späne oder Magnesium-Pulver.

Als Katalysatoren kommen bei der Durchführung der ersten Stufe des erfindungsgemäßen Verfahrens alle für derartige Grignard-Reaktionen üblichen Reaktionsbeschleuniger in Frage. Vorzugsweise verwendbar ist Iod.

Als Verdünnungsmittel dient bei der Durchführung der ersten Stufe des erfindungsgemäßen Verfahrens ein Gemisch aus Toluol und Tetrahydrofuran, in dem das Verhältnis von Toluol zu Tetrahydrofuran zwischen 65:35 und 95:5, vorzugsweise zwischen 70:30 und 90:10 Gewichtsteilen liegt.

Bei der Durchführung der zweiten Stufe des erfindungsgemäßen Verfahrens arbeitet man zweckmäßigerweise in Gegenwart desjenigen Verdünnungsmittels, das auch bei der Durchführung der ersten Stufe verwendet wird. Als Verdünnungsmittel fungiert daher im allgemeinen ein Gemisch aus Toluol und Tetrahydrofuran, in dem das Verhältnis von Toluol zu Tetrahydrofuran zwischen 65:35 und 95:5, vorzugsweise zwischen 70:30 und 90:10 Gewichtsteilen liegt. Es ist jedoch auch möglich, das Gemisch aus Toluol und Tetrahydrofuran ganz oder teilweise durch andere inerte Verdünnungsmittel zu ersetzen. Als derartige Verdünnungsmittel kommen inerte organische Solventien, wie aliphatische, alicyclische und aromatische Kohlenwasserstoffe in Betracht. Beispielhaft genannt seien Pentan, Hexan, Heptan, Cyclohexan, Methylcyclohexan, Xylol und Benzol sowie Gemische dieser Lösungsmittel.

Die Reaktionstemperaturen können bei der Durchführung des erfindungsgemäßen Verfahrens sowohl in der ersten als auch in der zweiten Stufe innerhalb eines größeren Bereiches variiert werden. In der ersten Stufe arbeitet man im allgemeinen bei Temperaturen zwischen 0 °C und 100 °C, vorzugsweise zwischen 20 °C und 50 °C. In der zweiten Stufe arbeitet man im allgemeinen bei Temperaturen zwischen 0 °C und 100 °C, vorzugsweise zwischen 0 °C und 30 °C.

Auch die Reaktionszeiten können im Falle des erfindungsgemäßen Verfahrens sowohl bei der Durchführung der ersten als auch der zweiten Stufe innerhalb eines größeren Bereiches variiert werden. Sie richten sich im allgemeinen nach der Menge der umzusetzenden Stoffe.

Das erfindungsgemäße Verfahren wird im allgemeinen unter Normaldruck durchgeführt. Es ist jedoch auch möglich, unter vermindertem oder erhöhtem Druck zu arbeiten.

Bei der Durchführung der ersten Stufe des erfindungsgemäßen Verfahrens setzt man das 2-Chlorbenzylchlorid der Formel (II) in Gegenwart eines Gemisches aus Toluol und Tetrahydrofuran mit einer äquivalenten Menge oder auch mit einem Überschuß an Magnesium sowie gegebenenfalls mit einer sehr kleinen Menge an Katalysator um. Die Mengen sind dabei so zu bemessen, daß auf 1 Mol an 2-Chlorbenzylchlorid der Formel (II) zwischen 1 und 5 Mol, vorzugsweise zwischen 1,05 und 3 Mol an Magnesium, zwischen 1 und 3 Mol, vorzugsweise zwischen 1,5 und 2,5 Mol an Tetrahydrofuran sowie gegebenenfalls zwischen 0,001 und 0,008 Mol an Katalysator vorhanden sind.

Auch bei der Durchführung der zweiten Stufe des erfindungsgemäßen Verfahrens kann das Verhältnis der Reaktionskomponenten innerhalb eines bestimmten Bereiches variiert werden. Dabei werden die Mengenverhältnisse so gewählt, daß auf 1 Mol an 1-Chlor-1-chloracetyl-cyclopropan der Formel (IV) im allgemeinen zwischen 1 und 2,5 Mol, vorzugsweise zwischen 1,1 und 1,7 Mol an Grignard-Verbindung der Formel (III) vorhanden sind.

Nach Beendigung der ersten Stufe wird gegebenenfalls enthaltenes, überschüssiges Magnesium nach üblichen Methoden abgetrennt. Die Entfernung des Magnesiums kann zum Beispiel durch Filtration oder Zentrifugieren erfolgen, oder auch dadurch geschehen, daß man nach dem Absetzen des Magnesiums die überstehende Lösung abdekantiert oder abpumpt.

Bei der Durchführung des erfindungsgemäßen Verfahrens geht man im einzelnen so vor, daß man zunächst zerkleinertes Magnesium im Gemisch mit einer geringen Menge an Verdünnungsmittel sowie gegebenenfalls im Gemisch mit dem Katalysator vorlegt, dann etwas 2-Chlor-benzylchlorid hinzugibt und nach dem Anspringen der Reaktion den verbleibenden Teil des 2-Chlor-benzylchlorids mit Verdünnungsmittel gemischt, langsam hinzutropft. Nach beendeter Umsetzung entfernt man gegebenenfalls vorhandenes, überschüssiges Magnesium und gibt dann 1-Chlor-1-chloracetyl-cyclopropan sowie gegebenenfalls zusätzliches Verdünnungsmittel zu der Grignard-Lösung hinzu. Die anschließende Aufarbeitung erfolgt nach üblichen Methoden. Im allgemeinen geht man so vor, daß man das Reaktionsgemisch entweder auf ein gekühltes Gemisch aus Wasser und Säure gibt oder auf gekühltes Wasser gießt und dann durch Hinzufügen von Säure neutralisiert oder schwach sauer stellt, anschließend die organische Phase abtrennt und nach dem Waschen mit Wasser einengt. Die Aufarbeitung kann jedoch auch so erfolgen, daß man nach beendeter Umsetzung zunächst auf Temperaturen zwischen 30 °C und 100 °C erhitzt, dann abkühlt, den dabei anfallenden Feststoff absaugt und das Filtrat einengt. Das nach der jeweiligen Aufarbeitung gewonnene Produkt kann gegebenenfalls nach üblichen Methoden weiter gereinigt werden.

In einer besonderen Variante kann die Grignard-Reaktion des erfindungsgemäßen Verfahrens in einer Wirbelschicht durchgeführt werden. Zu diesem Zweck kann man in einer Apparatur arbeiten, wie sie in Abbildung 1 schematisch dargestellt ist. In dieser Abbildung haben die angegebenen Ziffern die folgenden Bedeutungen:

1 = Reaktionsrohr
2 = Sieb am Eingang des Reaktionsrohres
3 = Sieb am Ausgang des Reaktionsrohres
4 = Untere Füllhöhe
5 = Obere Füllhöhe
6 = Vorratsbehälter
7 = Pumpe
8 = Ventil
9 = Vorratsbehälter
10 = Einfüllstutzen
11 = Temperaturmeßstelle
12 = Temperaturmeßstelle
13 = Reaktionsgefäß
14 = Vorratsbehälter
15 = Wärmeaustauscher
16 = Wärmeaustauscher

Zur Durchführung des erfindungsgemäßen Verfahrens in einem Wirbelschicht-Umlaufreaktor der in Abbildung 1 skizzierten Art geht man im allgemeinen in der nachstehend angegebenen Weise vor:

Das Reaktionsrohr 1, an dessen Eingang 2 und Ausgang 3 sich jeweils ein Sieb befindet, wird mit

zerkleinertem Magnesium bis zur Füllhöhe 4 gefüllt. Um das Anspringen der Grignard-Reaktion zu erleichtern, wird beim ersten Ansatz eine geringe Menge an Katalysator auf die Oberfläche des Magnesiums gegeben. Aus dem Vorratsbehälter 6 wird dann ein Gemisch aus Toluol und Tetrahydrofuran mit Hilfe der Pumpe 7 über das Ventil 8 langsam in das Reaktionsrohr 1 gedrückt, bis die Flüssigkeitsoberfläche die Füllhöhe 4 erreicht. Aus dem Vorratsbehälter 9, in dem sich ein Gemisch aus Toluol, Tetrahydrofuran und 2-Chlorbenzylchlorid befindet, wird mit einer Pipette eine kleine Menge an Gemisch entnommen und zum Starten der Grignard-Reaktion durch den Einfüllstutzen 10 in das Reaktionsrohr 1 gegeben. Sobald die Reaktion angesprungen ist, wird die Strömungsgeschwindigkeit des aus dem Vorratsbehälter 6 stammenden Lösungsmittelgemisches durch das Ventil 8 so eingestellt, daß das Magnesium im Reaktionsrohr 1 bis zur Höhe 5 aufgewirbelt wird. Gleichzeitig wird mit dem Eindosieren des Gemisches begonnen, das im Vorratsbehälter 9 enthalten ist. Mit Hilfe des Wärmeaustauschers 15 wird die Temperatur des umlaufenden Gemisches so gesteuert, daß sie an der Temperaturmeßstelle 11 bei der gewünschten Reaktionstemperatur liegt. An der Temperaturmeßstelle 12 ergibt sich ein jeweils um etwa 1 ° C höherer Wert. Nach Beendigung der Zugabe des Gemisches aus dem Vorratsbehälter 9 läßt man eine Weile unter weiterem Umpumpen nachreagieren und läßt dann den gesamten flüssigen Inhalt aus der Apparatur in das Reaktionsgefäß 13 einfließen. Dazu läßt man unter Kühlung und Rühren aus dem Vorratsbehälter 14 1-Chlor-1-chloracetyl-cyclopropan zutropfen. Nach beendeter Zugabe wird noch einige Zeit nachgerührt und dann aufgearbeitet, indem man mit einem Gemisch aus Wasser und Säure hydrolysiert, die Phasen trennt und die organische Phase einengt.

Bei allen folgenden Ansätzen, die im Wirbelschicht-Umlaufreaktor durchgeführt werden, erübrigt sich die erneute Zugabe von Katalysator. Das verbrauchte Magnesium wird jeweils ersetzt.

Die Umsetzung in der Wirbelschicht kann entweder chargenweise oder auch kontinuierlich durchgeführt werden.

Bei der Durchführung des erfindungsgemäßen Verfahrens entsteht 1-Chlor-2-(1-chlor-cyclopropyl)-3-(2-chlor-phenyl)-propan-2-ol der Formel (Ia) und/oder 2-(1-Chlor-cyclopropyl)-2-(2-chlor-benzyl)-oxiran der Formel (Ib). Die Substanz der Formel (Ia) ist im allgemeinen in einem höheren Anteil enthalten als die Verbindung der Formel (Ib). Das 1-Chlor-2-(1-chlor-cyclopropyl)-3-(2-chlor-phenyl)-propan-2-ol der Formel (Ia) kann aber durch Abspaltung von Halogenwasserstoff, zum Beispiel thermisch oder mit Hilfe von Basen, in das 2-(1-Chlor-cyclopropyl)-2-(2-chlor-benzyl)-oxiran der Formel (Ib) überführt werden.

Die nach dem erfindungsgemäßen Verfahren herstellbaren Produkte aus 1-Chlor-2-(1-chlor-cyclopropyl)-3-(2-chlor-phenyl)-propan-2-ol der Formel (Ia) und/oder 2-(1-Chlor-cyclopropyl)-2-(2-chlor-benzyl)-oxiran der Formel (Ib) sind wertvolle Zwischenprodukte zur Herstellung des fungizid wirksamen 2-(1-Chlor-cyclopropyl)-1-(2-Chlor-phenyl)-3-(1,2,4-triazol-1-yl)-propan-2-ols. So läßt sich das 2-(1-Chlor-cyclopropyl)-1-(2-chlor-phenyl)-3-(1,2,4-triazol-1-yl)-propan-2-ol der Formel

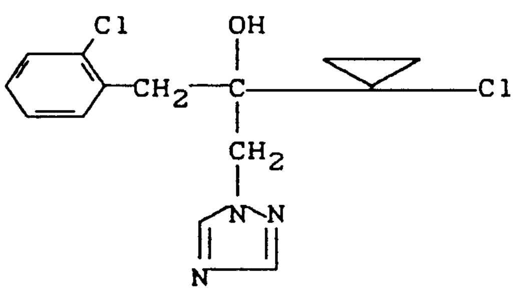

(V)

herstellen, indem man entweder 1-Chlor-2-(1-chlor-cyclopropyl)-3-(2-chlor-phenyl)-propan-2-ol der Formel

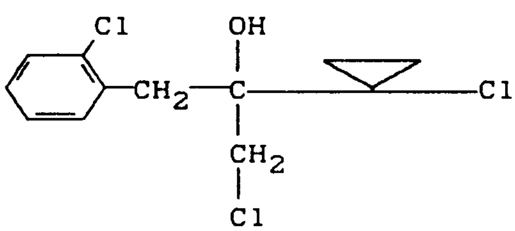

(Ia)

oder 2-(1-Chlor-cyclopropyl)-2-(2-chlor-benzyl)-oxiran der Formel

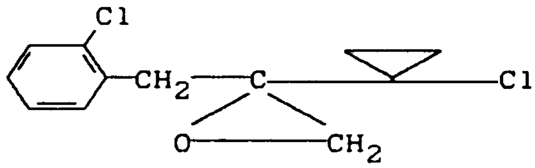

(Ib)

oder ein Gemisch der Substanzen der Formeln (Ia) und (Ib) mit 1,2,4-Triazol der Formel

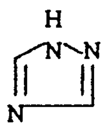

(VI)

in Gegenwart eines Säurebindemittels und in Gegenwart eines Verdünnungsmittels bei Temperaturen zwischen 0°C und 150°C umsetzt.

Die Durchführung des erfindungsgemäßen Verfahrens wird durch die folgenden Beispiele veranschaulicht.

## Herstellungsbeispiele

### Beispiel 1

Ein Gemisch aus 17 g (0,7 Mol) Magnesium-Spänen und 0,1 g Iod wird bei 20°C mit 42 g Toluol, 8 g Tetrahydrofuran und 1 g 2-Chlor-benzylchlorid versetzt. Nach dem Einsetzen der Reaktion wird ein Gemisch aus 97 g (insgesamt 0,61 Mol) 2-Chlor-benzylchlorid, 338 g Toluol und 67 g Tetrahydrofuran bei Temperaturen zwischen 50 und 55°C innerhalb von 5 Stunden eingetropft. Man läßt nach beendeter Zugabe noch 30 Minuten bei 50 bis 55°C nachreagieren, kühlt das Reaktionsgemisch dann auf 20°C ab und dekantiert vom nicht umgesetzten Magnesium ab. In das abdekantierte Reaktionsgemisch werden 87 g (0,54 Mol) 1-Chlor-1-chloracetyl-cyclopropan innerhalb von 45 Minuten bei Temperaturen zwischen 20 und 30°C eingetropft. Man läßt nach beendeter Zugabe noch 30 Minuten bei 20 bis 30°C nachreagieren und gibt das Reaktionsgemisch dann innerhalb von 30 Minuten bei Temperaturen zwischen 0°C und 20°C auf eine Lösung von 24 g konzentrierter Schwefelsäure in 170 g Wasser. Die organische Phase wird abgetrennt, zweimal mit je 100 g Wasser gewaschen und dann bei 80°C unter vermindertem Druck eingeengt. Man erhält auf diese Weise 156 g eines Produktes, das zu 44,2 % aus 1-Chlor-2-(1-chlor-cyclopropyl)-3-(2-chlor-phenyl)-propan-2-ol und zu 30,4 % aus 2-(1-Chlor-cyclopropyl)-2-(2-chlor-benzyl)-oxiran besteht. Bezogen auf eingesetztes 1-Chlor-1-chloracetyl-cyclopropan errechnet sich danach eine Ausbeute von 81,8 % der Theorie.

### Beispiel 2

Ein Gemisch aus 34 g (1,4 Mol) Magnesium-Spänen und 0,1 g Iod wird bei 20°C mit 26 g Toluol, 1 g Tetrahydrofuran und 1 g 2-Chlor-benzylchlorid versetzt. Nach dem Einsetzen der Reaktion wird ein Gemisch aus 97 g (insgesamt 0,61 Mol) 2-Chlor-benzylchlorid und 67 g Tetrahydrofuran bei Temperaturen zwischen 40 und 45°C innerhalb von 4,5 Stunden eingetropft. Während der ersten 15 Minuten der Zugabe gibt man gleichzeitig 158 g Toluol so hinzu, daß die Reaktionswärme zur Erwarmung des Lösungsmittels ausgenutzt wird. Man laßt nach beendeter Zugabe noch 30 Minuten bei 40 bis 45°C nachreagieren, kühlt das Reaktionsgemisch dann auf 20°C ab und dekantiert von nicht umgesetzten Magnesium ab. In das abdekantierte Reaktionsgemisch werden 78 g (0,48 Mol) 1-Chlor-1-chloracetyl-cyclopropan innerhalb von 60 Minuten bei Temperaturen zwischen 20 und 30°C eingetropft. Man läßt nach beendeter Zugabe noch 30 Minuten bei 20 bis 25°C nachreagieren und arbeitet auf, indem man das Reaktionsgemisch zunächst eine Stunde unter Rückfluß erhitzt, dann auf 20°C abkühlt und den ausgefallenen Feststoff absaugt. Das Filtrat wird bei 80°C unter vermindertem Druck einengt. Man erhält auf diese Weise 138 g eines Produktes, das zu 68,4 % aus 2-(1-Chlor-cyclopropyl)-2-(2-chlor-benzyl)-oxiran besteht. Bezogen auf eingesetztes 1-Chlor-1-chloracetyl-cyclopropan errechnet sich danach eine Ausbeute von 80,9 % der Theorie.

Beispiel 3

Ein Gemisch aus 68 g (2,8 Mol) Magnesium-Spänen und 0,1 g Iod wird bei 20° C mit 26 g Toluol, 1 g Tetrahydrofuran und 1 g 2-Chlor-benzylchlorid versetzt. Nach dem Einsetzen der Reaktion wird ein Gemisch aus 97 g (insgesamt 0,61 Mol) 2-Chlor-benzylchlorid und 69 g Tetrahydrofuran bei Temperaturen zwischen 40 und 45° C innerhalb von 4,5 Stunden eingetropft. Während der ersten 15 Minuten der Zugabe gibt man gleichzeitig 154 g Toluol so hinzu, daß die Reaktionswärme zur Erwärmung des Lösungsmittels ausgenutzt wird Man läßt nach beendeter Zugabe noch 30 Minuten bei 40 bis 45° C nachreagieren, kühlt das Reaktionsgemisch dann auf 20° C ab und dekantiert vom nicht umgesetzten Magnesium ab. In das abdekantierte Reaktionsgemisch werden 61 g (0,375 Mol) 1-Chlor-1-chloracetyl-cyclopropan innerhalb von 60 Minuten bei Temperaturen zwischen 20 und 25° C eingetropft. Man läßt nach beendeter Zugabe noch 30 Minuten bei 20 bis 25° C nachreagieren und gibt das Reaktionsgemisch dann innerhalb von 30 Minuten bei Temperaturen zwischen 0° C und 10° C auf eine Lösung von 24 g konzentrierter Schwefelsäure in 170 g Wasser. Die organische Phase wird abgetrennt, zweimal mit je 100 g Wasser gewaschen und dann bei 70 bis 75° C unter vermindertem Druck eingeengt. Man erhält auf diese Weise 130 g eines Produktes, das zu 55,1 % aus 1-Chlor-2-(1- chlor-cyclopropyl)-3-(2-chlor-benzyl)-propan-2-ol und zu 15,8 % aus 2-(1-Chlor-cyclopropyl)-2-(2-chlor-benzyl)- oxiran besteht. Bezogen auf eingesetztes 1-Chlor-1- chloracetyl-cyclopropan errechnet sich danach eine Ausbeute von 90,8 % der Theorie.

Beispiel 4

Ein Gemisch aus 190 g (7,8 Mol) Magnesium-Spänen und 0,5 g Iod wird bei 20° C mit 200 g Toluol, 10 g Tetrahydrofuran und 3 g 2-Chlor-benzylchlorid versetzt. Nach dem Einsetzten der Reaktion wird ein Gemisch aus 1160 g (insgesamt 7,2 Mol) 2-Chlor-benzylchlorid und 881 g Tetrahydrofuran bei Temperaturen zwischen 40 und 45° C innerhalb von 5 Stunden eingetropft. Während der ersten 30 Minuten der Zugabe gibt man gleichzeitig 2.200 g Toluol so hinzu, daß die Reaktionswärme zur Erwärmung des Lösungsmittels ausgenutzt wird. Man läßt nach beendeter Zugabe noch 30 Minuten bei 40 bis 45° C nachreagieren, kühlt das Reaktionsgemisch dann auf 20° C ab und dekantiert vom nicht umgesetzten Magnesium ab. In das abdekantierte Reaktionsgemisch werden 965 g (6 Mol) 1-Chlor-1-chloracetyl-cyclopropan innerhalb von einer Stunde bei Temperaturen zwischen 20 und 25° C eingetropft. Man läßt nach beendeter Zugabe noch 30 Minuten bei 20 bis 25° C nachreagieren und gibt das Reaktionsgemisch dann innerhalb von 30 Minuten bei Temperaturen zwischen 0° C und 10° C auf eine Lösung von 150 g konzentierter Schwefelsäure in 1700 g Wasser. Die organische Phase wird abgetrennt, zweimal mit je 500 g Wasser gewaschen und dann bei 80° C unter vermindertem Druck eingeengt. Man erhält auf diese Weise 1750 g eines Produktes, das zu 53,1 % aus 1-Chlor-2-(1-chlor-cyclopropyl)-3-(2-chlor-phenyl)-propan-2-ol und zu 22,8 % aus 2-(1-Chlor-cyclopropyl)-2-(2-chlor-benzyl)-oxiran besteht. Bezogen auf eingesetztes 1-Chlor-1-chloracetyl-cyclopropan errechnet sich danach eine Ausbeute von 82,7 % der Theorie.

Beispiel 5

Durchführung der Grignard-Reaktion des erfindungsgemäßen Verfahrens in einem Wirbelschicht-Umlaufreaktor des in Abbildung 1 skizzierten Typs.

Das Reaktionsrohr 1, an dessen Eingang 2 und Ausgang 3 sich jeweils ein Sieb befindet, wird mit 60 g Magnesium-Spänen (1-3 mm) bis zur Füllhöhe 4 gefüllt. Um das Einsetzen der Grignard-Reaktion zu erleichtern, wird eine Spatelspitze Iod auf die Oberfläche des Magnesiums gegeben. Aus dem Vorratsbehälter 6 wird dann ein Gemisch aus 750 g Toluol und 20 g Tetrahydrofuran mit Hilfe der Pumpe 7 über das Ventil 8 langsam in das Reaktionsrohr 1 gedrückt, bis die Flüssigkeitsoberfläche die Füllhöhe 4 erreicht. Aus dem Vorratsbehälter 9, in dem sich ein Gemisch aus 100 g Toluol, 130 g Tetrahydrofuran und 161 g 2-Chlor-benzylchlorid befindet, werden mit einer Pipette 5 ml an Gemisch entnommen und zum Starten der Grignard-Reaktion durch den Einfüllstutzen 10 in das Reaktionsrohr 1 gegeben. Das Einsetzen der Grignard-Reaktion erkennt man an örtlicher Erwärmung und am Verschwinden der durch das Iod verursachten Braunfärbung. Sobald die Reaktion angesprungen ist, wird die Strömungsgeschwindigkeit des aus dem Vorratsbehälter 6 stammenden Lösungsmittelgemisches durch das Ventil 8 so eingestellt, daß das Magnesium im Reaktionsrohr 1 bis zur Höhe 5 aufgewirbelt wird. Gleichzeitig wird mit dem Eindosieren des Gemisches begonnen, das im Vorratsbehälter 9 enthalten ist. Die Zugabe erfolgt innerhalb von 5 Stunden. Dabei wird die Temperatur des umlaufenden Gemisches mit Hilfe des Wärmeaustauschers 15 so gesteuert,

daß sie an der Temperaturmeßstelle 11 bei 35 bis 37°C liegt. An der Temperaturmeßstelle 12 ergibt sich ein um 1°C höherer Wert. Nach Beendigung der Zugabe des Gemisches aus dem Vorratsbehälter 9 läßt man noch 90 Minuten unter weiterem Umpumpen nachreagieren und läßt dann den gesamten flüssigen Inhalt aus der Apparatur in das Reaktionsgefäß 13 einfließen. Dazu läßt man bei 10 bis 15°C unter Rühren aus dem Vorratsbehälter 14 innerhalb von 30 Minuten 100 g 1-Chlor-1-chloracetyl-cyclopropan zutropfen. Nach beendeter Zugabe wird noch 30 Minuten bei 20°C nachgerührt und dann aufgearbeitet, indem man mit einem Gemisch aus Wasser und Essigsäure hydrolysiert. Die organische Phase wird abgetrennt und unter vermindertem Druck eingeengt. Es verbleiben 224 g eines Öles, das gemäß Gaschromatogramm 125,0 g 1-Chlor-2-(1-chlor-cyclopropyl)-3-(2-chlorphenyl)-propan-2-ol und 10,9 g 2-(1-Chlor-cyclopropyl)-2-(2-chlor-benzyl)-oxiran enthält. Bezogen auf eingesetztes 1-Chlor-1-chlor-acetyl-cyclopropan errechnet sich danach eine Ausbeute von 82,3 % der Theorie.

Beispiel 6

Durchführung der Grignard-Reaktion des erfindungsgemäßen Verfahrens in einem kontinuierlich arbeitenden Wirbelschicht-Umlaufreaktor.

Das Reaktionsrohr, in dem sich 500 g Magnesium-Späne zwischen den Sieben befinden, wird mit einem Gemisch aus 2-Chlorbenzylchlorid, Toluol und Tetrahydrofuran der gewünschten Zusammensetzung gefüllt. Mit einer Pumpe wird das Gemisch mit einer Strömungsgeschwindigkeit von 200 Litern pro Stunde im Kreislauf gepumpt, so daß das Magnesium im Reaktionsrohr aufgewirbelt wird. Durch Kühlung mit einem Wärmeaustauscher wird die Temperatur in der Wirbelschicht konstant auf 35°C gehalten. Außerdem wird das Reaktionsgemisch in der gesamten Apparatur mit Stickstoff überlagert.

Danach wird mit einer Dosierpumpe kontinuierlich ein Gemisch, das zu
13,7 Gewichtsprozent aus 2-Chlor-benzylchlorid,
12,7 Gewichtsprozent aus Tetrahydrofuran und
73,5 Gewichtsprozent aus Toluol
besteht, mit einer Geschwindigkeit von 2,8 kg pro Stunde von unten in die Wirbelschicht im Reaktionsrohr eingepumpt. Über einen Überlauf fließt gleichzeitig ein Gemisch, das die gebildete Grignard-Verbindung enthält, mit etwa der gleichen Geschwindigkeit von 2,8 kg pro Stunde in einen Rührreaktor. Durch einen Kugelhahn werden stündlich 57 g Magnesium-Späne in das Reaktionsrohr nachgefüllt.

Im Rührreaktor befinden sich 100 Minuten nach Inbetriebnahme der Apparatur 4,8 kg. Der Überlauf wird nun geschlossen, während das Gemisch aus dem Reaktionsrohr mit Hilfe der Dosierpumpe weiter in der Apparatur zirkuliert wird. Dabei kontinuierlich entstehende Grignard-Verbindung wird in einen weiteren Vorratsbehälter geleitet und dort gelagert bis sie in dem dann entleerten Rührreaktor zur weiteren Reaktion eingesetzt wird.

Aus einem zusätzlichen Vorratsbehälter werden 510 g 1-Chlor-1-chloracetyl-cyclopropan innerhalb von 30 Minuten bei 20 bis 25°C in den Rührreaktor eindosiert, in dem sich die Grignard-Verbindung im Gemisch mit Toluol und Tetrahydrofuran befindet. Das Reaktionsgemisch wird nach beendeter Zugabe noch 15 Minuten bei 20 bis 25°C nachgerührt, dann abgelassen und bis zur Aufarbeitung in einem getrennten Gefäß gesammelt. Danach wird der Überlauf erneut geöffnet, so daß wieder Grignard-Verbindung in den Rührreaktor gelangt. Außerdem wird auch die im Vorratsbehälter gelagerte Grignard-Verbindung in den Rührreaktor geleitet, so daß dort eine weitere Umsetzung mit 1-Chlor-1-chloracetyl-cyclopropan erfolgt.

Zur Aufarbeitung wird das Reaktionsgemisch mit einem Gemisch aus konzentrierter Schwefelsäure und Wasser versetzt. Die organische Phase wird abgetrennt und unter vermindertem Druck bei erhöhter Temperatur eingeengt. Dabei verbleiben 20 g eines Öles, das gemäß Gaschromatogramm zu 16,9 % aus 1-Chlor-2-(1-chlor-cyclopropyl)-3-(2-chlor-phenyl)-propan-2-ol und zu 52,4 % aus 2-(1-Chlor-cyclopropyl)-2-(2-chlorbenzyl)-oxiran besteht. Bezogen auf eingesetztes 1-Chlor-1-chloracetyl-cyclopropan errechnet sich danach eine Ausbeute von 92,1 % der Theorie.

Vergleichsbeispiel 1

Verwendung von Methyl-tert.-butylether als Verdünnungsmittel.

Ein Gemisch aus 7 g (0,29 Mol) Magnesium-Spänen, 40 g Methyl-tert.-Butylether und 0,1 g Iod wird bei 20°C mit 3 g 2-Chlor-benzylchlorid versetzt und dann auf 30°C erwärmt. Nach dem Einsetzen der Reaktion gibt man zunächst weitere 80 g Methyl-tert.-butylether hinzu. Danach wird bei 50°C eine Lösung von 42 g (insgesamt 0,28 Mol) 2-Chlor-benzylchlorid in 80 g Methyl-tert.-butylether innerhalb von 3 Stunden eingetropft. Man läßt nach beendeter Zugabe noch eine Stunde bei 50°C nachreagieren, kühlt das

Reaktionsgemisch auf 20 ° C ab und dekantiert vom nicht ungesetzten Magnesium ab. Dieses abdekantierte Reaktionsgemisch gibt man innerhalb von einer Stunde unter Rühren in eine Lösung aus 25 g (0,155 Mol) 1-Chlor-1-chlor-acetyl-cyclopropan in 40 g Methyl-tert.-butylether. Das Reaktionsgemisch wird nach beendeter Zugabe noch 3 Stunden bei 20 ° C gerührt, mit Eiswasser versetzt und mit verdünnter Schwefelsäure neutralisiert.

Die organische Phase wird abgetrennt, zweimal mit je 100 g Wasser gewaschen und dann bei 70 ° C unter vermindertem Druck eingeengt. Man erhält auf diese Weise 53,4 g eines Produktes, das zu 40,3 % aus 1-Chlor-2-(1-chlor-cyclopropyl)-3-(2-chlor-phenyl)-propan-2-ol besteht. Bezogen auf eingesetztes 1-Chlor-1-chloracetyl-cyclopropan errechnet sich danach eine Ausbeute von 50,1 % der Theorie.

Vergleichsbeispiel 2

Verwendung von Methyl-tert.-amyl-ether als Verdünnungsmittel.

Ein Gemisch aus 40 g (1,65 Mol) Magnesium-Spänen, 85 g Methyl-tert.-amyl-ether und 0,1 g Iod wird bei 20 ° C mit 5 g 2-Chlor-benzylchlorid versetzt und dann auf 60 ° C erwärmt. Nach dem Einsetzen der Reaktion gibt man zunächst weitere 700 g Methyl-tert.-amyl-ether hinzu. Danach werden 196 g (insgesamt 1,25 Mol) 2-Chlor-benzylchlorid bei Temperaturen zwischen 65 und 70 ° C innerhalb von 4 Stunden eingetropft. Anschließend tropft man bei 45 bis 50 ° C innerhalb von 2 Stunden 195 g (1,2 Mol) 1-Chlor-1-chloracetyl-cyclopropan hinzu. Das Reaktionsgemisch wird nach beendeter Zugabe noch 2 Stunden bei 30 bis 35 ° C gerührt und dann bei 5 bis 10 ° C innerhalb von einer Stunde mit 350 g Wasser und 80 g Eisessig versetzt. Die organische Phase wird abgetrennt, mit 200 g Wasser gewaschen und dann bei 70 ° C unter vermindertem Druck eingeengt. Man erhält auf diese Weise 350 g eines Produktes, das zu 51,5 % aus 1-Chlor-2-(1-chlor-cyclopropyl)-3-(2-chlor-phenyl)-propan-2-ol besteht. Bezogen auf eingesetztes 1-Chlor-1-chloracetyl-cyclopropan errechnet sich danach eine Ausbeute von 53,7 % der Theorie.

Vergleichsbeispiel 3

Verwendung von 2-Chlor-benzylbromid zur Herstellung der Grignard-Komponente.

Ein Gemisch aus 17 g (0,7 Mol) Magnesium-Spänen und 0,1 g Iod wird bei 20 ° C mit 40 g Toluol sowie mit 4 g eines Gemisches, das aus 75 g Tetrahydrofuran und 127 g (0,6 Mol) 2-Chlor-benzylbromid erhalten wurde, versetzt. Nach dem Einsetzen der Reaktion werden innerhalb von 4 Stunden die restlichen 198 g des Gemisches aus Tetrahydrofuran und 2-Chlor-benzylbromid hinzugetropft, wobei die Temperatur des Reaktionsgemisches zwischen 35 und 45 ° C gehalten wird. Während der ersten 15 Minuten der Zugabe gibt man gleichzeitig 140 g Toluol so hinzu, daß die Reaktionswärme zur Erwärmung des Lösungsmittels ausgenutzt wird. Man läßt nach beendeter Zugabe noch 30 Minuten bei 40 bis 45 ° C nachreagieren, kühlt das Reaktionsgemisch dann auf 20 ° C ab und dekantiert vom nicht umgesetzten Magnesium ab. In das abdekantierte Reaktionsgemisch werden 82 g (0,5 Mol) 1-Chlor-1-chloracetyl-cyclopropan innerhalb von 1,5 Stunden bei Temperaturen zwischen 10 und 20 ° C eingetropft. Man läßt nach beendeter Zugabe noch 30 Minuten bei 20 bis 30 ° C nachreagieren und gibt das Reaktionsgemisch dann innerhalb von 30 Minuten bei Temperaturen zwischen 0 ° C und 20 ° C auf eine Lösung von 15 g konzentrierter Schwefelsäure in 200 g Wasser. Die organische Phase wird abgetrennt, zweimal mit je 100 g Wasser gewaschen und dann bei 80 ° C unter vermindertem Druck eingeengt. Man erhält auf diese Weise 162 g eines Produktes, das zu 38,9 % aus 1-Chlor-2-(1-chlor-cyclopropyl)-3-(2-chlor-phenyl)-propan-2-ol und zu 13,5 % aus 2-(1-Chlor-cyclopropyl)-2-(2-chlor-benzyl)-oxiran besteht. Bezogen auf eingesetztes 1-Chlor-1-chloracetyl-cyclopropan errechnet sich danach eine Ausbeute von 62,9 % der Theorie.

Vergleichsbeispiel 4

Verwendung eines Verdünnungsmittels, das zu gleichen Teilen aus Toluol und Tetrahydrofuran besteht.

Ein Gemisch aus 17 g (0,7 Mol) Magnesium-Spänen und 0,1 g Iod wird bei 20 ° C mit 10 g Toluol, 10 g Tetrahydrofuran und 1 g 2-Chlor-benzylchlorid versetzt. Nach dem Einsetzen der Reaktion wird ein Gemisch aus 97 g (insgesamt 0,61 Mol) 2-Chlor-benzylchorid,240 g Toluol und 240 g Tetrahydrofuran bei Temperaturen zwischen 35 und 45 ° C innerhalb von 5 Stunden eingetropft. Man läßt nach beendeter Zugabe noch 30 Minuten bei 30 bis 40 ° C nachreagieren, kühlt das Reaktionsgemisch dann auf 20 ° C ab und dekantiert vom nicht umgesetzten Magnesium ab, In das abdekantierte Reaktionsgemisch werden 87 g (0,54 Mol) 1-Chlor-1-chloracetyl-cyclopropan innerhalb von 40 Minuten bei Temperaturen zwischen 20 und 30 ° C eingetropft. Man läßt nach beendeter Zugabe noch 30 Minuten bei 30 bis 35 ° C nachreagieren und gibt das Reaktionsgemisch dann innerhalb von 30 Minuten bei Temperaturen zwischen 0 ° C und 10 ° C auf

eine Lösung von 30 g Eisessig in 120 g Wasser. Die organische Phase wird abgetrennt, mit 100 g Wasser gewaschen und dann bei 70 ° C unter vermindertem Druck eingeengt.

Man erhält auf diese Weise 166 g eines Produktes, das zu 5,6 % aus 1-Chlor-2-(1-chlor-cyclopropyl)-3-(2-chlorphenyl)-propan-2-ol und zu 2,4 % aus 2-(1-Chlorcyclopropyl)-2-(2-chlor-benzyl)-oxiran besteht. Bezogen auf eingesetztes 1-Chlor-1-chloracetyl-cyclopropan errechnet sich danach eine Ausbeute von 9,2 % der Theorie.

Vergleichsbeispiel 5

Verwendung von Tetrahydrofuran als alleiniges Verdünnungsmittel.

Ein Gemisch aus 17 g (0,7 Mol) Magnesium-Spänen und 0,1 g Iod wird bei 20 ° C mit 20 g Tetrahydrofuran und 1 g 2-Chlor-benzylchlorid versetzt, Nach dem Einsetzen der Reaktion wird eine Lösung von 97 g (insgesamt 0,61 Mol) 2-Chlorbenzylchlorid in 250 g Tetrahydrofuran bei Temperaturen zwischen 40 und 45 ° C innerhalb von 4 Stunden eingetropft. Man läßt nach beendeter Zugabe noch eine Stunde bei 30 bis 40 ° C nachreagieren, kühlt das Reaktionsgemisch dann auf 20 ° C ab und dekantiert vom nicht umgesetzten Magnesium ab. In das abdekantierte Reaktionsgemisch werden 10 g (0,06 Mol) 1-Chlor-1-chloracetyl-cyclopropan innerhalb von 30 Minuten bei Temperaturen zwischen 20 und 25 ° C eingetropft. Man läßt nach beendeter Zugabe noch 30 Minuten bei 20 bis 30 ° C nachreagieren und tropft dann innerhalb von 30 Minuten bei 0 ° C bis 10 ° C eine Lösung von 30 g konzentrierter Schwefelsäure in 150 g Wasser in das Reaktionsgemisch. Man schüttelt bei 20 ° C mit 170 g Toluol aus und engt die organische Phase bei 70 ° C unter vermindertem Druck ein, Man erhält auf diese Weise 82 g eines Produktes, in dem das 1-Chlor-2-(1-chlor-cyclopropyl)-3-(2-chlor-phenyl)-propan-2-ol und das 2-(1-Chlor-cyclopropyl)-2-(2-chlor-benzyl)-oxiran zu weniger als 1 % enthalten sind.

Verwendungsbeispiel

Herstellung der Verbindung der Formel

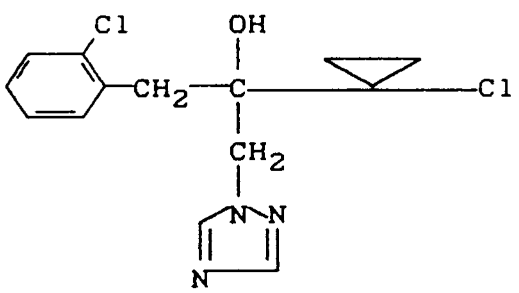

(V)

Eine Lösung von 218 g eines Gemisches, das zu 53,8 % (0,42 Mol) aus 1-Chlor-2-(1-chlor-cyclopropyl)-3-(2-chlor-phenyl)-propan-2-ol und zu 8,9 % (0,08 Mol) aus 2-(1-Chlor-cyclopropyl)-2-(2-chlor-benzyl)-oxiran besteht, in 160 g Methanol wird bei 20 ° C mit 45 g (0,65 Mol) 1,2,4-Triazol versetzt, Danach gibt man 138 g (1 Mol) wasserfreies Kaliumcarbonat bei Temperaturen zwischen 30 und 35 ° C innerhalb einer Stunde hinzu, Anschließend erwärmt man das Reaktionsgemisch auf 70 ° C und läßt 4,5 Stunden bei dieser Temperatur reagieren. Das Reaktionsgemisch wird auf 20 ° C abgekühlt und unter Rühren mit 500 g Wasser versetzt. Nach 30 Minuten dekantiert man die obere, wäßrige Phase ab und nimmt den Rückstand in 150 g Methylenchlorid und 250 g Wasser auf, Man rührt 15 Minuten bei 20 ° C,saugt den ausgefallenen Feststoff ab und wäscht mit 75 g Methylenchlorid nach, Das Filtrat wird nach Abtrennung der wäßrigen Phase bei 80 ° C unter vermindertem Druck eingeengt. Der verbleibende Rückstand wird in einem Gemisch aus 165 g Petrolether und 25 g Isopropanol aufgenommen, Man erwärmt das Gemisch auf 60 ° C, bis eine klare Lösung entstanden ist, und kühlt dann langsam innerhalb von 2 Stunden auf 10 ° C ab. Der dabei auskristallisierende Feststoff wird abgesaugt, mit einem gekühlten Gemisch aus 70 g Petrolether und 15 g Isopropanol gewaschen und bei 50 ° C unter vermindertem Druck getrocknet. Man erhält auf diese Weise 92 g eines Produktes, das zu 96,3 % aus 2-(1-Chlor-cyclopropyl)-1-(2-chlor-phenyl)-3-(1,2,4-triazol-1-yl)-propan-2-ol besteht, Danach errechnet sich eine Ausbeute von 56,8 % der Theorie.

## Patentansprüche

1. Verfahren zur Herstellung von 1-Chlor-2-(1-chlor-cyclopropyl)-3-(2-chlor-phenyl)-propan-2-ol der Formel

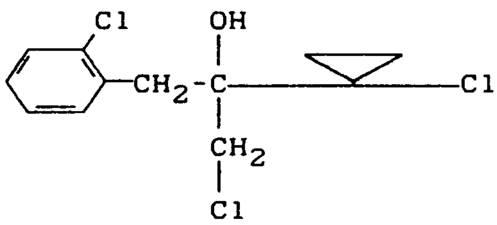

(Ia)

und/oder 2-(1-Chlor-cyclopropyl)-2-(2-chlor-benzyl)-oxiran der Formel

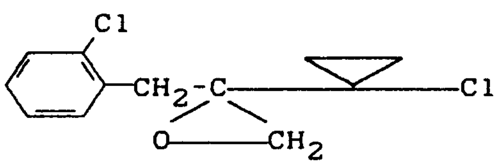

(Ib)

dadurch gekennzeichnet, daß man
a) 2-Chlor-benzylchlorid der Formel

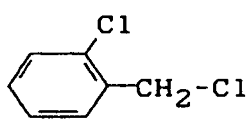

(II)

mit zerkleinertem Magnesium, gegebenenfalls in Gegenwart eines Katalysators, in Gegenwart eines Gemisches von Toluol und Tetrahydrofuran, in dem das Verhältnis von Toluol zu Tetrahydrofuran zwischen 65:35 und 95:5 Gewichtsteilen liegt, bei Temperaturen zwischen 0°C und 100°C umsetzt, wobei das Verhältnis von 2-Chlor-benzylchlorid der Formel (II) zu Tetrahydrofuran so bemessen ist, daß auf 1 Mol an 2-Chlor-benzylchlorid der Formel (II) zwischen 1 und 3 Mol an Tetrahydrofuran vorhanden sind,

anschließend gegebenenfalls noch vorhandenes Magnesium abtrennt und

b) das entstandene Grignard-Reagenz der Formel

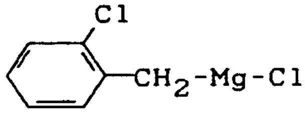

(III)

mit 1-Chlor-1-chloracetyl-cyclopropan der Formel

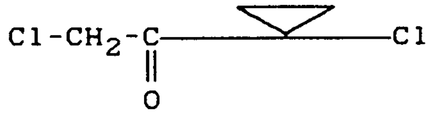

(IV)

in Gegenwart eines Gemisches von Toluol und Tetrahydrofuran, in dem das Verhältnis von Toluol zu Tetrahydrofuran zwischen 65:35 und 95:5 Gewichtsteilen liegt, sowie gegebenenfalls in Gegenwart eines zusätzlichen inerten Verdünnungsmittels bei Temperaturen zwischen 0 ° C und 100 ° C umsetzt.

**2.** Verfahren gemäß Anspruch 1, dadurch gekennzeichnet, daß man bei der Durchführung der ersten Stufe Magnesium-Späne einsetzt.

**3.** Verfahren gemäß Anspruch 1, dadurch gekennzeichnet, daß man bei der Durchführung der ersten Stufe Iod als Katalysator einsetzt.

**4.** Verfahren gemäß Anspruch 1, dadurch gekennzeichnet, daß man bei der Durchführung der ersten Stufe als Verdünnungsmittel ein Gemisch aus Toluol und Tetrahydrofuran einsetzt, in dem das Verhältnis von Toluol zu Tetrahydrofuran zwischen 70:30 und 90:10 Gewichtsteilen liegt.

**5.** Verfahren gemäß Anspruch 1, dadurch gekennzeichnet, daß man bei der Durchführung der zweiten Stufe in Gegenwart eines zusätzlichen Verdünnungsmittels arbeitet.

**6.** Verfahren gemäß Anspruch 1, dadurch gekennzeichnet, daß man bei der Durchführung der ersten Stufe bei Temperaturen zwischen 20 und 50 ° C und bei der Durchführung der zweiten Stufe bei Temperaturen zwischen 0 und 30 ° C arbeitet.

**7.** Verfahren gemäß Anspruch 1, dadurch gekennzeichnet, daß man bei der Durchführung der ersten Stufe auf 1 Mol an 2-Chlor-benzylchlorid der Formel (II) zwischen 1,5 und 2,5 Mol an Tetrahydrofuran einsetzt.

**8.** Verfahren gemäß Anspruch 1, dadurch gekennzeichnet, daß man bei der Durchführung der ersten Stufe in einer Wirbelschicht arbeitet.

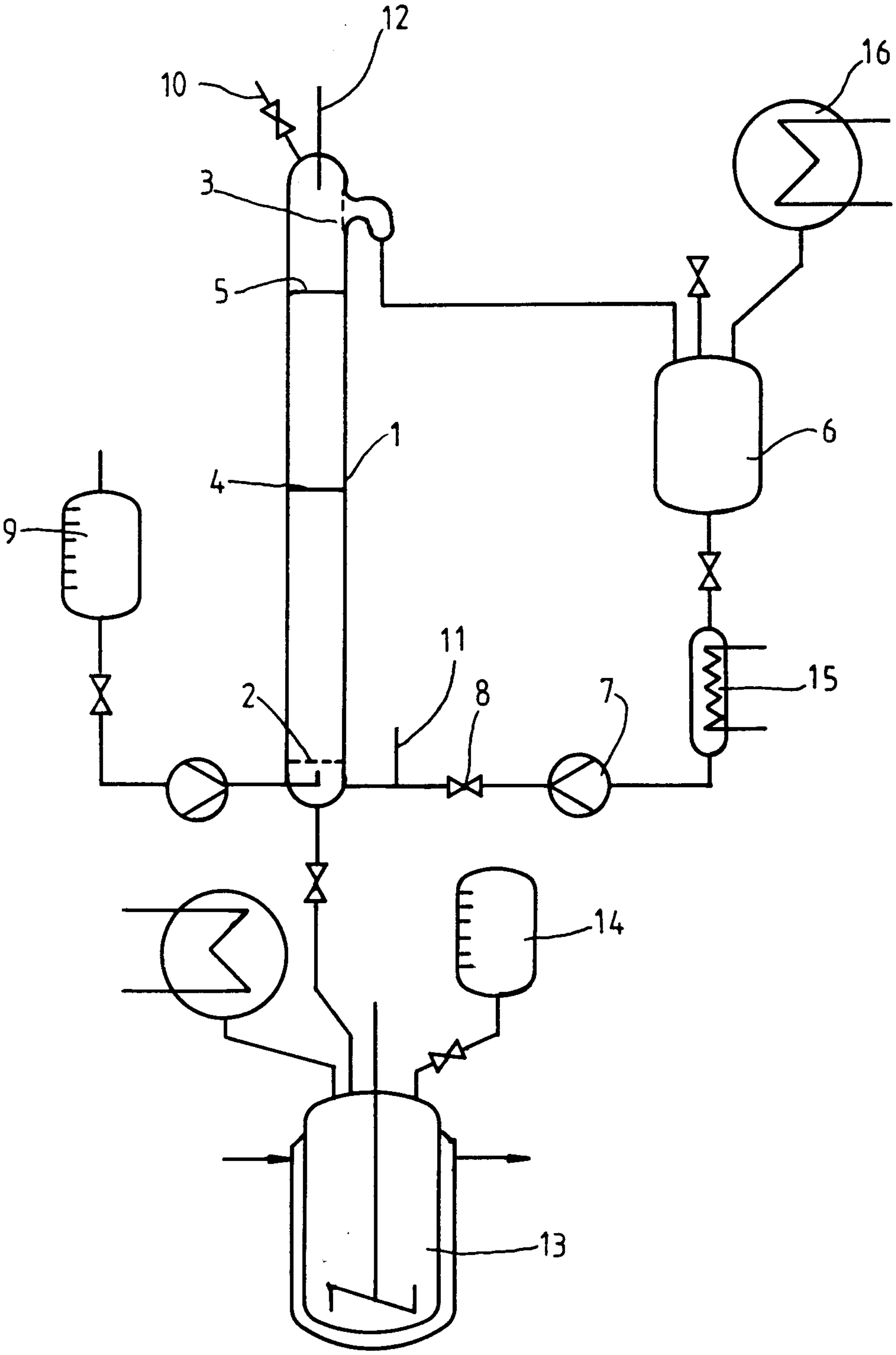


FIG.1

Europäisches Patentamt

# EUROPÄISCHER RECHERCHENBERICHT

Nummer der Anmeldung

**EP 91 11 3811**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br><br>Y | GB-A-870 415 (IMPERIAL CHEMICAL INDUSTRIES)<br>* das ganze Dokument *<br><br>– – – | 1,3,4-8<br><br>1-8 | C 07 C 29/40<br>C 07 C 33/50<br>C 07 D 303/08<br>C 07 F 3/02 |
| Y,D | EP-A-0 297 345 (BAYER AG)<br>* Seiten 7-12 *<br>– – – | 1-8 | |
| A | EP-A-0 047 594 (IMPERIAL CHEMICAL INDUSTRIES PLC)<br>* Seiten 7,8,24,25; Ansprüche 1,16,29 *<br>– – – | 1 | |
| A | DE-A-2 149 087 (NATIONAL PATENT DEVELOPMENT CORP.)<br>* Seiten 24-26 *<br>– – – | 1,2 | |
| A,P | EP-A-0 415 247 (BASF AG)<br>* Seiten 4,5 *<br>– – – | 1 | |
| A | BULLETIN DE LA SOCIETE CHIMIQUE DE FRANCE Band 45, Nr. 4, August 1929, Seiten 1091-1095; H. GILMAN et al.: "L'action d'une ébullition prolongée sur quelques halogénures organomagnésiens"<br>* Seiten 1092-1095 *<br>– – – – – | 1 | |
| | | | **RECHERCHIERTE SACHGEBIETE (Int. Cl.5)** |
| | | | C 07 C 29/00<br>C 07 C 33/00<br>C 07 D 303/00<br>C 07 F 3/00 |

Der vorliegende Recherchenbericht wurde für alle Patentansprüche erstellt

| Recherchenort | Abschlußdatum der Recherche | Prüfer |
|---|---|---|
| Berlin | 13 Dezember 91 | RUFET J.M.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument